# EUROPÄISCHE PATENTSCHRIFT

(11) **EP 1 863 917 B1**
(45) Veröffentlichungstag und Bekanntmachung des Hinweises auf die Patenterteilung: **21.09.2016**
(21) Anmeldenummer: 06723138.1
(22) Anmeldetag: 28.02.2006
(51) Int. Cl.: C12P 7/40, C12P 7/42, C08F 2/00, C08F 4/30

(54) **VERFAHREN ZUR HERSTELLUNG EINES WASSERABSORBIERENDEN POLYMERGEBILDES BASIEREND AUF ACRYLSÄURE**
METHOD FOR PRODUCING WATER-ABSORBENT POLYMER STRUCTURES BASED ON ACRYLIC ACID
PROCEDE DE FABRICATION DE STRUCTURES POLYMERES ABSORBANT L'EAU A BASE D'ACIDE ACRYLIQUE

(30) Priorität: 28.02.2005 DE 102005009584
(43) Veröffentlichungstag der Anmeldung: 12.12.2007
(73) Patentinhaber: Evonik Degussa GmbH, 45128 Essen (DE)
(72) Erfinder: BUB, Günther, 45772 Marl (DE); MOSLER, Jürgen, 44577 Castrop-Rauxel (DE); SABBAGH, Andreas, 48249 Dülmen (DE); KUPPINGER, Franz-Felix, 45768 Marl (DE); FURNO, Franck Dr., 17799 Krefeld (DE)
(74) Vertreter: Lang, Arne
(86) Internationale Anmeldenummer: PCT/EP2006/001830
(87) Internationale Veröffentlichungsnummer: WO 2006/092271

(56) Entgegenhaltungen:
- EP-A1- 1 302 485
- WO-A-01/16346
- WO-A-02/42418
- WO-A-02/056812
- WO-A-2004/037903
- WO-A-2005/054488
- WO-A1-01/44145
- WO-A2-03/082795
- US-A- 4 962 027
- SANSEVERINO J ET AL: "Detection of acrylic acid formation in Megashaera elsdenii in the presence of 3-butynoic acid", APPLIED MICROBIOLOGY AND BIOTECHNOLOGY, vol. 30, 1989, pages 239-242,
- DANNER H ET AL: "Biotechnological Production of Acrylic Acid from Biomass", APPLIED BIOCHEMISTRY AND BIOTECHNOLOGY, vol. 70-72, 1998, pages 887-894,

## Beschreibung

Die vorliegende Erfindung betrifft ein Verfahren zur Herstellung von Polymeren, vorzugsweise wasserabsorbierenden Polymergebilden, durch radikalische Polymerisation von Acrylsäure, die durch dieses Verfahren erhältlichen wasserabsorbierenden Polymergebilde, wasserabsorbierende Polymergebilde, welche zu mindestens 25 Gew.% auf teilneutralisierter Acrylsäure basieren, einen Verbund, ein Verfahren zur Herstellung eines Verbundes, den durch dieses Verfahren erhältlichen Verbund, die Verwendung von Acrylsäure zur Herstellung von Polymeren, vorzugsweise zur Herstellung wasserabsorbierender Polymergebilde, eine Vorrichtung zur Herstellung von Acrylsäure, ein Verfahren zur Herstellung von Acrylsäure sowie die durch dieses Verfahren erhältliche Acrylsäure.

An die Reinheit von Monomeren, welche zur Herstellung polymerer Verbindungen eingesetzt werden, werden hohe Anforderungen gestellt. Dieses gilt insbesondere dann, wenn es sich bei den Polymeren um sogenannte Superabsorber handelt. Diese Polymere sind in der Lage, wässrige Flüssigkeiten unter Bildung eines Hydrogels zu absorbieren und damit zu binden. Superabsorber finden daher insbesondere in Hygieneartikeln wie Windeln, Inkontinenzeinlagen, Damenbinden und dergleichen zur Absorption von Körperflüssigkeiten Verwendung. Einen umfassenden Überblick über Superabsorber, ihre Anwendung und ihre Herstellung geben F. L. Buchholz und A. T. Graham (Herausgeber) in "Modern Superabsorbent Polymer Technology", Wiley-VCH, New York, 1998.

Zur Herstellung der superabsorbierenden Polymere wird üblicherweise ein Acrylsäure eingesetzt, welche durch katalytische Gasphasenoxidation von Propylen zu Acrolein, das dann in einer weiteren katalytischen Gasphasenoxidation zu Acrylsäure, anschließende Absorption des gasförmigen Reaktionsgemisches in Wasser, Destillation der so erhaltenen wässrigen Acrylsäurelösung unter Erhalt einer Roh-Acrylsäure und weitere Aufreinigung der Roh-Acrylsäure mittels Destillation oder Kristallisation als Rein-Acrylsäure erhalten wird.

Nachteilig bei diesem Verfahren zur Herstellung von Acrylsäure ist. dass die in beiden Stufen angewandten Temperaturen zwischen 300 und 450°C zur Bildung von Oligomeren und weiteren unerwünschten Crackprodukten führten. Dieses hat zur Folge, dass eine unerwünscht hohe Menge an höher als die Acrylsäure siedenden Verbindungen oder an Verbindungen, die nur schwer von der Acrylsäure zu trennen sind, wie etwa Essigsäure, anfallen. Diese Verbindungen müssen in der Regel destillativ von der Acrylsäure abgetrennt werden, was wiederum zu einer weiteren thermischen Belastung der Acrylsäure und der damit verbundenen Bildung von Dimeren und Oligomeren führt. Ein hoher Gehalt an Acrylsäuredimeren oder Acrylsäureoligomeren ist jedoch nachteilig, da diese Dimeren oder Oligomeren bei der Herstellung von Superabsorbern durch radikalische Polymerisation von Acrylsäure in Gegenwart von Vernetzern in das Polymerrückrad eingebaut werden. Bei der im Anschluss an die Polymerisation erfolgenden Nachbehandlung der Oberfläche der Polymerpartikel, beispielsweise im Rahmen einer Oberflächennachvemetzung, werden die einpolymerisierten Dimere jedoch unter Bildung von β-Hydroxypropionsäure gespalten, die unter den Nachvernetzungsbedingungen unter Bildung von Acrylsäure dehydratisiert wird. Ein hoher Gehalt an dimerer Acrylsäure in der zur Herstellung der Superabsorber eingesetzten Acrylsäure führt daher dazu, dass der Gehalt an Acrylsäuremonomeren bei einer thermischen Behandlung der Polymere, wie sie bei der Nachvernetzung erfolgt ansteigt.

Da die löslichen Anteile, insbesondere die Acrylsäuremonomere in den superabsorbierenden Polymeren Hautreizungen hervorrufen können, setzt ein Einsatz dieser Polymere in Hygieneartikeln einen besonders geringen Gehalt an extrahierbaren Bestandteilen voraus.

Auch andere, oftmals toxische Verbindungen sind in der durch die katalytische Gasphasenoxidation erhältlichen Acrylsäure noch enthalten. Zu diesen Verunreinigungen gehören insbesondere Aldehyde, die sich störend auf den Polymerisationsverlauf auswirken, mit der Folge, dass die Polymere noch beachtliche Mengen an löslichen Bestandteilen beinhalten.

Auf herkömmliche Weise aus Propylen hergestellte Acrylsäure enthält nicht unerhebliche Mengen an Doppelbindungen aufweisenden Ketonen, insbesondere an Protoanemonin (PTA). Diese Verbindung kann bei einem Kontakt mit der Haut Vergiftungserscheinungen, wie etwa Rötung, Juckreiz oder Blasenbildung, hervorrufen. Auf Acrylsäure basierende Superabsorber, welche hohe Mengen an PTA als lösliche Bestandteile beinhalten, sind daher aus dermatologischer Sicht bedenklich. Außerdem stört PTA die Polymerisation, wie dies etwa in der US-A-2002/0120085 beschrieben ist. Dieses führt zum Erhalt von superabsorbierenden Polymeren mit weniger guten Saug-, Transport- und Rückhalteeigenschaften von Körperflüssigkeiten, so dass bei Verwendung von derartigen superabsorbierenden Polymeren in Hygieneartikeln wie Windeln oder Damenbinden ein schlechterer Tragekomfort beispielsweise durch "Leakage" auftritt.

Im Stand der Technik sind bereits einige Verfahren beschrieben worden, mit denen der Gehalt an den vorstehend genannten Verbindungen, insbesondere von Aldehyden oder PTA, in der durch Gasphasenoxidation von Propylen erhaltenen Acrylsäure reduziert werden kann.

DE-A-101 38 150 schlägt zur Verminderung des Aldehyd-Anteils in der Acrylsäure vor, diese mit einem Aldehydfänger in Kontakt zu bringen, um die Aldehyde in Hochsiedende Verbindungen zu überführen, die dann mittels Destillation abgetrennt werden können.

Zur Entfernung des PTAs werden im Stand der Technik verschiedene Verfahren vorgeschlagen, wie etwa die Zugabe eines Salzes der salpetrigen Säure, von Stickstoffoxid oder von Nitrobenzol (JP 81-41614) oder die Zugabe eines oder mehrerer para-Phenylendiamine (EP-A-567 207) zur Acrylsäure.

Der Nachteil der vorstehend beschriebenen Verfahren zur Verminderung des Anteils an Aldehyden und Ketonen in Acrylsäure besteht jedoch unter anderem darin, dass, sofern der Gehalt an Verunreinigungen in der Acrylsäure nicht genau bekannt ist, diese Reagenzien zum Zwecke einer möglichst vollständigen Entfernung der Verunreinigungen aus der Acrylsäure im Überschuss eingesetzt werden müssen. zum einen der Acrylsäure reaktive Reagenzien zugesetzt werden müssen. Der nicht umgesetzte Teil dieser Reagenzien muss anschließend wieder entfernt werden. Nicht entfernte Reagenzien sind in den aus einer solchen Acrylsäure erhaltenen Superabsorbent als lösliche Bestandteile enthalten, die bei einem Einsatz der Superabsorber in Hygieneartikeln in Kontakt mit der Haut des Hygieneartikelträgers kommen können. Weiterhin lassen sich mit den aus dem Stand der Technik bekannten Verfahren zur Entfernung von Aldehyden und Ketonen aus Acrylsäure diese Verunreinigungen nur selten vollständig entfernen.

Neben den Nachteilen, die auf Verunreinigungen in der zur Herstellung von Superabsorbern eingesetzten Acrylsäure zurückzuführen sind, weisen herkömmliche Superabsorber auch den Nachteil auf, dass sie, sofern sie nicht zumindest anteilig natürliche Polymere, wie etwa Zellulose, beinhalten, kaum auf nachwachsenden Rohstoffen basieren. Zwar ist es gelungen, viele der in Hygieneartikeln, insbesondere in Wegwerfwindeln, eingesetzten Komponenten aus biologischen Ausgangsstoffen herzustellen, doch ist ein Ersatz der auf vernetzten Polyacrylaten basierenden Superabsorber durch natürliche, superabsorbierende Polymere, wie etwa durch vernetzte, derivatisierte Stärke oder Zellulose, in der Regel mit signifikanten Einbußen hinsichtlich der Absorbereigenschaften verbunden. Dieses führt meist dazu, dass, um auch nur annährend die gleichen Absorbereigenschaften in einem Hygieneartikel zu erreichen, wesentlich mehr dieser auf natürlichen Polymeren basierenden Absorber eingesetzt werden müssen. Dieses ist nachteilig, weil die Hygieneartikel dadurch voluminöser und schwerer werden, was den Tragekomfort deutlich einschränkt und zu einem größeren Abfallvolumen führt, das neben mehr Deponieraum bzw. höherem Verbrennungsaufwand mehr Transportkapazitäten für die Abfallentsorgung notwendig macht. All dieses wirkt sich nachteilig auf die Umweltverträglichkeit der auf natürlichen Polymeren basierenden Absorber aus.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, Polymere, insbesondere Superabsorber, bereitzustellen, welche einen besonders geringen Gehalt an extrahierbaren, gegebenenfalls toxischen Bestandteilen aufweisen.

Weiterhin lag der vorliegenden Erfindung die Aufgabe zugrunde, Polymere, insbesondere Superabsorber, bereitzustellen, die besonders umweltverträglich sind und dennoch über hervorragende Anwendungseigenschaften verfügen. So galt es insbesondere Superabsorber mit einer verbesserten Umweltverträglichkeit bei gleich bleibend guten Absorbereigenschaften zu schaffen.

Zudem war es eine Aufgabe der vorliegenden Erfindung, die Umweltverträglichkeit der die erfindungsgemäßen Polymere beinhaltenden Weiterverarbeitungsprodukte, wie Verbunde allgemein und Hygieneartikel im Besonderen, zu verbessern, ohne dass die gewünschten Funktionen, wie Saugfähigkeit, Tragekomfort und einfache Herstellbarkeit, dieser Weiterverarbeitungsprodukte darunter leiden.

Auch lag der vorliegenden Erfindung die Aufgabe zugrunde, ein Verfahren zur Herstellung derartiger Polymere und der zur deren Herstellung geeigneten Monomere anzugeben, wobei dieses Verfahren möglichst ohne den Einsatz reaktiver Verbindungen zur Entfernung von Verunreinigungen aus den zur Herstellung der Polymere eingesetzten Monomere auskommen soll.

Zudem lag eine Aufgabe der vorliegenden Erfindung darin, ein Verfahren und eine Vorrichtung zur Herstellung der Monomere bzw. der Polymere vorzuschlagen, die mit einem möglichst geringen Umrüstaufwand in bestehende großtechnische Fertigungsverfahren und -vorrichtungen integriert werden kann.

Einen Beitrag zur Lösung der vorstehend genannten Aufgaben leistet ein Verfahren zur Herstellung von Polymeren durch radikalische Polymerisation von Acrylsäure, wobei das Polymer ein wasserabsorbierendes Polymer ist, welches erhältlich ist durch ein Verfahren umfassend die folgenden Verfahrensschritte:
i) Polymerisation der Acrylsäure in Gegenwart eines Vernetzers unter Ausbildung eines Polymergels;
ii) gegebenenfalls Zerkleinerung des Polymergels;
iii) Trocknung des gegebenenfalls zerkleinerten Polymergels unter Erhalt wasserabsorbierender Polymergebilde, sowie
iv) gegebenenfalls Oberflächennachbehandlung der wasserabsorbierenden Polymergebilde, gemäß Anspruch 1, welches folgenden Verfahrensschritt umfasst:
   Spaltung eines organischen Materials mittels eines Enzyms oder mindestens eines Bestandteils von einem Enzym, durch Fermentation oder durch aus Zellen isolierten, gegebenenfalls auf einem Substrat immobilisierten Enzymen oder mindestens eines Bestandteils von Enzymen erfolgt, das organische Material zu mindestens 75 Gew.-% auf einem Kohlenhydrat basiert, wobei als organisches Material Malzextrakt, Hafermehl oder Milchpulver, reine, definierte Kohlenhydrate, insbesondere Polysaccharide, wie Raffinose, Stärke, Zellulose, Glykogen oder Dextrine, Disaccharide, wie Saccharose, Laktose oder Maltose, und Monosaccharide, vorzugsweise Hexosen wie Galaktose, Xylose, Glukose, Galaktose oder Fruktose, oder aber Zuckeralkohole eingesetzt werden, wobei durch die Spaltung des Kohlenhydrates β-Hydroxypropionsäure erhalten wird und wobei das Syntheseverfahren zur Herstellung der Acrylsäure neben der enzymatischen Spaltung des Kohlenhydrates unter

Erhalt von β-Hydroxypropionsäure als Verfahrensschritt a) und einen Verfahrensschritt b) umfasst:
katalytische Dehydratisierung der β-Hydroxypropionsäure unter Erhalt von Acrylsäure,
wobei als Katalysator ein mit einer anorganischen Säure in Kontakt gebrachter, porenförmiger Trägerkörper eingesetzt wird und der porenförmige Trägerkörper zu mindestens 90 Gew.-% auf einem Siliziumoxid basiert und eine Oberfläche in einem Bereich von 0,005 bis 450 m²/g aufweist.

Unter "Spaltung eines organischen Materials mittels eines Enzyms oder mittels eines Bestandteils von einem Enzym" wird erfindungsgemäß vorzugsweise ein Verfahren verstanden, bei dem ein organisches Material entweder mittels Mikroorganismen, welche in Form intakter, funktionsfähiger Zellen vorliegen, oder mittels aus Mirkoorganismen isolierten Enzymen gespalten werden. Die isolierten Enzyme könnten dabei in einem geeigneten Reaktionsmedium gelöst sein. Denkbar ist aber auch, die Enzyme auf einem Substrat, beispielsweise auf der Oberfläche einer porösen Matrix zu immobilisieren und das Reaktionsmedium dann an dem Substrat entlang bzw. durch die poröse Matrix hindurchströmen zu lassen.

Der Begriff "Spaltung" umfasst dabei sowohl eine Spaltung eines gegebenenfalls polymeren organischen Materials, wie etwa Zellulose oder Stärke, in einzelne Oligomere bzw. Monomere als auch die weitere Spaltung der Monomere in kleinere Fragmente.

Die wasserabsorbierenden Polymergebilde basieren vorzugsweise auf:
(α1) 20-99,999 Gew.-%, bevorzugt 55-98,99 Gew.-% und besonders bevorzugt 70-98,79 Gew.-% Acrylsäure,
(α2) 0-80 Gew.-%, vorzugsweise 0-44,99 Gew.-% und besonders bevorzugt 0,1-44,89 Gew.-% polymerisierten, monoethylenisch ungesättigten, mit der Acrylsäure copolymerisierbaren Monomeren,
(α3) 0,001-5 Gew.-%, vorzugsweise 0,01-3 Gew.-% und besonders bevorzugt 0,01-2,5 Gew.-% eines oder mehrerer Vernetzer,
(α4) 0-50 Gew.-%, vorzugsweise 0-25 Gew.-% und besonders bevorzugt 0,1-5 Gew.-% eines wasserlöslichen Polymers,
(α5) 0-20 Gew.-%, vorzugsweise 2,5-15 Gew.-% und besonders bevorzugt 5-10 Gew.-% Wasser, sowie
(α6) 0-20 Gew.-%, vorzugsweise 0-10 Gew.-% und besonders bevorzugt 0,1-8 Gew.-% eines oder mehrerer Hilfsmittel, wobei die Summe der Gewichtsmengen (α1) bis (α6) 100 Gew.-% beträgt.

Die Acrylsäuremonomere (α1) können teilweise oder vollständig, bevorzugt teilweise neutralisiert sein. Vorzugsweise sind Acrylsäuremonomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen, deren Offenbarung hiermit als Referenz eingeführt wird. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Als monoethylenisch ungesättigte, mit Acrylsäure copolymerisierbare Monomere (α2) sind Acrylamide und Methacrylamide bevorzugt.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino-(meth)acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit Acrylsäure copolymerisierbare Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl-(meth)acrylat oder Butyl(meth)-acrylat, sowie Methylpolyethylenglykol-(meth)acrylat, Methylpolyethylenglykol-allylether, Vinylacetat, Styrol und Isobutylen bevorzugt.

Als Vernetzer (α3) werden vorzugsweise diejenigen Verbindungen eingesetzt, die in der WO 2004/037903 A1 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi-(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als wasserlösliche Polymere (α4) können in den Polymergebilden wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsmittel (α6) sind vorzugsweise Stellmittel, Geruchsbinder, oberflächenaktive Mittel oder Antioxidatien sowie diejenigen Additive, die zur Herstellung der Polymergebilde eingesetzt wurden (Initiatoren usw.) in den Polymergebilden enthalten.

In einer besonderen Ausführungsform der erfindungsgemäßen wasserabsorbierenden Polymergebilde basieren diese zu mindestens 50 Gew.-%, vorzugsweise zu mindestens 70 Gew.-% und darüber hinaus bevorzugt zu mindestens 90 Gew.-% auf Acrylsäuremonomeren, die zu mindestens 20 Mol-%, besonders bevorzugt zu mindestens 50 Mol-% und darüber hinaus bevorzugt in einem Bereich von 60 bis 85 Mol-% neutralisiert ist.

Erfindungsgemäß bevorzugte Polymergebilde sind Fasern, Schäume oder Teilchen, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500, bevorzugt 2 bis 500 und besonders bevorzugt 5 bis 100 mm und einen Durchmesser im Bereich von 1 bis 200, bevorzugt 3 bis 100 und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm aufweisen. Weiterhin ist es bevorzugt, dass die Polymerteilchen zu mindestens 50 Gew.-%, vorzugsweise zu mindestens 75 Gew.-% auf Partikeln mit einer Partikelgröße in einem Bereich von 300 bis 600 µm basieren.

Genauere Einzelheiten zur Herstellung von auf Acrylsäure basierenden wasserabsorbierenden Polymergebilden sind F. L. Buchholz und A. T. Graham (Herausgeber) in "Modern Superabsorbent Polymer Technology", Wiley-VCH, New York, 1998 zu entnehmen. Der Offenbarungsgehalt dieser Druckschrift hinsichtlich der Herstellung von auf Acrylsäure basierenden wasserabsorbierenden Polymeren, insbesondere hinsichtlich der einzusetzenden Monomere, der Polymerisationsbedingungen, der Aufarbeitung der Polymergele sowie der Oberflächenmodifizierugsmöglichkeiten wir hiermit als Referenz eingeführt und bildet einen Teil der Offenbarung der vorliegenden Erfindung.

Als organisches Material kann in dem erfindungsgemäßen Verfahren jedes Material eingesetzt werden, das unter der katalytischen Mitwirkung von Enzymen, beispielsweise im Rahmen eines Fermentationsprozesses, letztendlich unter Bildung von 3-Hydroxyprionsäure (β-Hydroxypropionsäure) gespalten werden kann, wobei diese Spaltung gegebenenfalls in mehreren Schritten erfolgen kann. Als organisches Material kommen zum Beispiel natürliche Gemische in Frage, wie Malzextrakt, Hafermehl oder Milchpulver, oder aber reine, definierte Kohlenhydrate, insbesondere Polysaccharide, zum Beispiel Raffinose, Stärke, Zellulose, Glykogen oder Dextrine, Disaccharide, zum Beispiel Saccharose, Laktose oder Maltose, und Monosaccharide, vorzugsweise Hexosen wie zum Beispiel Galaktose, Xylose, Glukose, Galaktose oder Fruktose, oder aber Zuckeralkohole, wobei Glucose als Kohlenstoffquelle besonders bevorzugt ist. Gemäß einer besondern Ausführungsform des erfindungsgemäßen Verfahrens basiert das organische Material zu mindestens 75 Gew.-%, besonders bevorzugt zu mindestens 85 Gew.-% und am meisten bevorzugt zu mindestens 95 Gew.-% auf gegebenenfalls polymerisierter Hexosen, vorzugsweise polymerisierter Glukose.

Weiterhin ist es erfindungsgemäß bevorzugt, dass das organische Material ein natürlich vorkommendes, organisches Material ist. Unter einem natürlich vorkommenden, organischen Material wird dabei vorzugsweise ein Material verstanden, welche innerhalb der letzten 4.000 Jahre, besonders bevorzugt innerhalb der letzten 1.000 Jahre, darüber hinaus bevorzugt innerhalb der letzten 10 Jahre und am meisten bevorzugt innerhalb der letzten 12 Monate auf der Erde auf natürlichem Weg, unter Ausnutzung der Photosynthese, durch Biosynthese (Anabolismus) entstanden ist.

Die Spaltung des organischen Materials führt gemäß einer besonderen Ausführungsform des erfindungsgemäßen Verfahrens zur Bildung von β-Hydroxypropionsäure, wobei es in diesem Zusammenhang bevorzugt ist, dass als organisches Material Glukose oder Xylose beinhaltende Materialien eingesetzt werden und die Spaltung der Glukose oder der Xylose mittels eines Fermentationsprozesses erfolgt. Dabei werden Mikroorganismen, welche in der Lage sind, geeignete organische Ausgangsstoffe unter Bildung von β-Hydroxypropionsäure zu spalten, in einem geeigneten Kulturmedium inkubiert, so dass sie bis zu einer gewünschten Zelldichte unter Bildung von β-Hydroxypropionsäure heranwachsen. Allgemeine Informationen für eine großtechnische Verfahrensweise können beispielsweise dem "Manual of Industrial Microbiology and Biotechnology, 2. Auflage, A.L. Demain und J. E. Davies (Herausgeber), ASM-Press, sowie "Principles of Fermentation Technology", P.F. Stanbury und A. Whitaker (Herausgeber), Pregamon, entnommen werden. Die Offenbarung dieser Druckschriften hinsichtlich der allgemeinen Vorgehensweise bei der Fermentation wird hiermit als Referenz eingeführt und bildet einen Teil der Offenbarung der vorliegenden Erfindung.

Üblicherweise wird bei einem Fermentationsprozess eine in einem Fermentationstank befindliche Nährlösung beinhaltend Kohlenstoffquellen, Stickstoffquellen, Salze sowie weitere, für die Vermehrung von Mikroorganismen notwenigen Substanzen mit geeigneten Mirkoorganismen beimpft. Anschließend werden die Mirkoorganismen bis zum Erreichen einer bestimmten Dichte in diesem Nährmedium kultiviert. Anschließend wird ein Teil der so erhaltenen Brühe in einem zweiten Fermentationstank, in dem ebenfalls ein geeignetes Nährmedium vorgelegt wird, überführt, wobei der zweite Fermentationstank vorteilhafterweise größer ist als der erste Fermentationstank. In diesem zweiten Fermentationstank wachsen die Mirkoorganismen dann bis zum Erreichen einer gewünschten Zelldichte oder bis zum Erreichen einer gewünschten β-Hydroxypropionsäure-Konzentration im Nährmedium. Ein solches Fermentationsverfahren kann auch kontinuierlich durchgeführt werden, wie dies beispielsweise in DE-A-197 18 608 für die Herstellung von Milchsäure beschrieben ist. Hinsichtlich der zur Herstellung von β-Hydroxypropionsäure aus Kohlenhydraten einzusetzenden Mirkoorganismen kann der WO-A-02/42418 entnommen werden, deren Offenbarungsgehalt hinsichtlich der Herstellung von β-Hydroxypropionsäure aus Kohlenhydraten mittels Fermentation hiermit als Referenz eingeführt wird und einen Teil der Offenbarung der vorliegenden Erfindung bildet.

Nachdem im Verlaufe der Fermentation ausreichende Mengen an β-Hydroxypropionsäure gebildet worden sind, kann die β-Hydroxypropionsäure durch jedes dem Fachmann bekannte Reinigungsverfahren isoliert werden. So können beispielsweise Sedimentations-, Filtrations- oder Zentrifugationsverfahren eingesetzt werden, um die Mikroorganismen abzutrennen. Aus der von Mikroorganismen befreiten, β-Hydroxypropionsäure-haltigen Nährlösung kann die Hydroxypropionsäure durch Extraktion, Destillation oder Ionen-Austausch isoliert werden.

Die Aufreinigung der β-Hydroxypropionsäure aus der Nährlösung erfolgt gemäß einer besonderen Ausgestaltung des erfindungsgemäßen Verfahrens kontinuierlich, wobei es in diesem Zusammenhang weiterhin bevorzugt ist, auch die Fermentation kontinuierlich durchzuführen, so dass der gesamte Prozess von der enzymatischen Spaltung des organischen Materials unter Bildung von β-Hydroxypropionsäure bis zur Aufreinigung der β-Hydroxypropionsäure aus der Fermentationsbrühe kontinuierlich durchgeführt werden kann. Zur kontinuierlichen Aufreinigung der β-Hydroxypropionsäure aus der Fermentationsbrühe wird diese kontinuierlich über eine Vorrichtung zur Abtrennung der bei der Fermentation eingesetzten Mikroorganismen, vorzugsweise über einen Filter mit einer Ausschlussgröße in einem Bereich von 20 bis 200 kDa geführt, in dem eine Fest-/Flüssig-Trennung stattfindet. Denkbar ist auch der Einsatz einer Zentrifuge, einer geeigneten Sedimentationsvorrichtung oder eine Kombination dieser Vorrichtungen, wobei es besonders bevorzugt ist, zumindest einen Teil der Mikroorganismen zunächst durch Sedimentation abzutrennen und anschließend die von den Mikroorganismen teilweise befreite Fermentationsbrühe einer Ultrafiltrations- oder Zentrifugationsvorrichtung zuzuführen. Das Retentat kann gemäß einer besonderen Ausgestaltung des erfindungsgemäßen Verfahrens wieder in den zweiten Fermentationstank zurückgeführt werden. Alternativ kann das Retentat abgeführt und gegebenenfalls verworfen werden.

Das hinsichtlich seines β-Hydroxypropionsäure-Anteils angereicherte Fermentationserzeugnis wird nach der Abtrennung der Mikroorganismen einer vorzugsweise mehrstufigen Trennanlage zugeführt. In dieser Trennanlage sind mehrere hintereinander geschaltete Trennstufen vorgesehen, aus denen jeweils RückführLeitungen ausmünden, die zum zweiten Fermentationstank zurückgeführt sind. Weiterhin führen aus den jeweiligen Trennstufen Ableitungen heraus. Die einzelnen Trennstufen können nach dem Prinzip der Elektrodialyse, der Umkehrosmose, der Ultrafiltration oder der Nanofiltration arbeiten. In der Regel handelt es sich um Membran-Trenneinrichtungen in den einzelnen Trennstufen. Die Auswahl der einzelnen Trennstufen ergibt sich aus Art und Umfang der Gärungs-Nebenprodukte und Substratreste.

Neben der Abtrennung der β-Hydroxypropionsäure mittels Elektrodialyse, Umkehrosmose, Ultrafiltration oder Nanofiltration, in deren Verlauf als Endprodukt eine wässrige β-Hydroxypropionsäure-Lösung erhalten wird, kann die β-Hydroxypropionsäure auch durch Extraktionsverfahren aus der von Mikroorganismen befreiten Fermentationslösung abgetrennt werden, wobei in diesem Fall letztendlich die reine β-Hydroxypropionsäure erhalten werden kann. Zur Abtrennung der β-Hydroxypropionsäure durch Extraktion können der Fermentationslösung beispielsweise Ammoniumverbindungen oder Amine zugesetzt werden, um ein Ammoniumsalz der β-Hydroxypropionsäure zu bilden. Dieses Ammoniumsalz kann dann aus der Fermentationslösung abgetrennt werden, in dem ein organisches Extraktionsmittel zugesetzt und die so erhaltene Mischung anschließend erhitzt wird, wodurch das Ammoniumsalz sich in der organischen Phase anreichert. Aus dieser Phase kann die β-Hydroxypropionsäure dann unter Erhalt der reinen β-Hydroxypropionsäure beispielsweise durch weitere Extraktionsschritte isoliert werden. Genauere Einzelheiten bezüglich dieses Trennverfahrens sind der WO-A-02/090312 zu entnehmen, deren Offenbarungsgehalt hinsichtlich der Abtrennung von β-Hydroxypropionsäure aus Fermentationslösungen hiermit als Referenz eingeführt wird und einen Teil der Offenbarung der vorliegenden Anmeldung bildet.

Je nach Art und Weise der Abtrennung der β-Hydroxypropionsäure aus der Fermentationslösung wird entweder eine wässrige β-Hydroxypropionsäure-Lösung, beinhaltend 2 bis 90 Gew.-%, vorzugsweise 7,5 bis 50 Gew.-% und besonders bevorzugt 10 bis 25 Gew.-% an β-Hydroxypropionsäure, oder aber reine β-Hydroxypropionsäure erhalten.

In dem erfindungsgemäßen Verfahrens zur Herstellung von Polymeren mittels radikalischer Polymerisation von Acrylsäure umfasst das Syntheseverfahren der Acrylsäure neben dem vorstehend beschriebenen Verfahrensschritt der enzymatischen Spaltung eines organischen Materials unter Bildung von β-Hydroxypropionsäure einen weiteren Verfahrensschritt, bei dem die β-Hydroxypropionsäure katalytisch unter Bildung von Acrylsäure dehydratisiert wird. Dabei kann in dem Dehydratisierungsschritt entweder die aus der Fermenationslösung isolierte, reine β-Hydroxypropionsäure oder aber die bei der Aufarbeitung der Fermenationslösung isolierte wässrige β-Hydroxypropionsäure-Lösung eingesetzt werden, wobei diese gegebenenfalls noch vor der Dehydratisierung beispielsweise durch Destillation, gegebenenfalls in Gegenwart eines geeigneten Schleppmittels, aufkonzentriert wird.

Die Dehydratisierung kann grundsätzlich in flüssiger Phase oder in der Gasphase durchgeführt werden. Weiterhin ist es erfindungsgemäß bevorzugt, dass die Dehydratisierung in Gegenwart eines Katalysators erfolgt, wobei die Art des eingesetzten Katalysators davon abhängig ist, ob eine Gasphasen- oder eine Flüssigphasenreaktion durchgeführt wird.

Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x. in Kontakt steht. Als Träger x. kommen alle dem Fachmann als geeignet erscheinenden Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und in einem Bereich von 0,1 bis 1,5 ml/g besonders bevorzugt. Zudem ist es bevorzugt, dass die als Träger x. geeigneten Feststoffe eine Oberfläche im Bereich von 0,001 bis 1000 m² /g, vorzugsweise im Bereich von 0,005 bis 450 m²/g und darüber hinaus bevorzugt im Bereich von 0,01 bis 300 m²/g nach BET-Test gemäß DIN 66131 aufweisen. Als Träger für den Dehydratisierungskatalysator kann zum einen ein Schüttgut, das einen mittleren Teilchendurchmesser im Bereich von 0,1 bis 40 mm, vorzugsweise im Bereich von 1 bis 10 mm und darüber hinaus bevorzugt im Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x., die auch Dehydratisierungskatalysatoreigenschaften aufweisen können, eignen sich insbesondere natürliche oder synthetische silikatische Stoffe, wie insbesondere Mordenit, Montmorillonit, saure Zeolithe; mit ein-, zwei oder mehrbasigen anorganischen Säuren, insbesondere Phosphorsäure, oder saure Salze anorganischer Säuren belegte Trägerstoffe, wie oxidische oder silikatische Stoffe, beispielsweise Al₂O₃, TiO₂; Oxide und Mischoxide, wie beispielsweise Gamma-Al₂O₃ und ZnO-Al₂O₃- Mischoxide der Heteropolysäuren.

Es entspricht einer erfindungsgemäßen Ausführungsform, dass der Träger x. mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x. als auch als Dehydratisierungskatalysator wirkende Verbindungsklasse beinhalten Silicium-Aluminium- Phosphoroxide. Bevorzugte basische sowohl als Dehydratisierungskatalysator als auch als Träger x. fungierende Stoffe beinhalten Alkali, Erdalkali, Lanthan, Lanthanoide oder eine Kombination von mindestens zwei davon in ihrer oxidischen Form. Derartige saure oder basische Dehydratisierungskatalysatoren sind sowohl bei der Degussa AG als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x. durch Eintauchen bzw. Imprägnieren immobilisiert werden.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

Zudem ist es bevorzugt, dass in dem erfindungsgemäßen Verfahren ein Dehydratisierungskatalysator mit einem H₀-Wert im Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von -3 bis -8,2 eingesetzt wird. Der H₀-Wert entspricht der Säurefunktion nach Hammett und lässt sich durch die sogenannte Amintritation und Verwendung von Indikatoren oder durch Absorption einer gasförmigen Base ermitteln - siehe *"*Studies in Surface Science and Catalytics", vol. 51, 1989: "New solid Acids and Bases, their catalytic Properties", K. Tannabe et al. Weitere Einzelheiten zur Herstellung von Acrolein aus Glycerin sind weiterhin DE 42 38 493 C1 zu entnehmen.

Gemäß einer besonderen Ausführungsform des erfindungsgemäßen Verfahrens wird als saurer Feststoffkatalysator ein mit einer anorganischen Säure, vorzugsweise mit Phosphorsäure oder mit Supersäuren wie etwa sulfatisiertem oder phosphatisiertem Zirkoniumoxid in Kontakt gebrachter porenförmiger Trägerkörper eingesetzt, der vorzugsweise zu mindestens 90 Gew.-%, darüber hinaus bevorzugt zu mindestens 95 Gew.-% und am meisten bevorzugt zu mindestens 99 Gew.-% auf einem Siliziumoxid, vorzugsweise auf SiO₂, basiert. Das in Kontakt bringen des porenförmigen Trägerkörpers mit der anorganischen Säure erfolgt vorzugsweise durch das Imprägnieren des Trägerkörpers mit der Säure, wobei diese vorzugsweise in einer Menge in einem Bereich von 10 bis 70 Gew.-%, besonders bevorzugt in einem Bereich 20 bis 60 Gew.-% und darüber hinaus bevorzugt in einem Bereich von 30 bis 50 Gew.-%, bezogen auf das Gewicht des Trägerkörpers, mit diesem in Kontakt gebracht und anschließend getrocknet wird. Im Anschluss an die Trocknung wird der Trägerkörper zur Fixierung der anorganischen Säure erhitzt, vorzugsweise auf eine Temperatur in einem Bereich von 300 bis 600 °C, darüber hinaus bevorzugt in einem Bereich von 400 bis 500 °C.

Gemäß einer besonderen Ausführungsform des erfindungsgemäßen Verfahrens wird die Dehydratisierung in der Gasphase durchgeführt. Dabei können übliche Apparaturen, wie sie dem Fachmann für Gasphasenreaktion bekannt sind, beispielsweise Röhrenreaktoren, eingesetzt werden. Besonders bevorzugt ist der Einsatz von Rohrbündel-Wärmeaustauschern sowie von Reaktoren, welche Thermobleche als Wärmeaustauscher beinhalten.

Gemäß einer Ausführungsform der Gasphasendehydratisierung wird reine β-Hydroxypropionsäure in einen Reaktor umfassend einen der vorstehend genannten Festbettkatalysatoren eingebracht. Gemäß einer anderen Ausführungsform wird die β-Hydroxypropionsäure in Form einer wässrigen Lösung beinhaltend 2 bis 80 Gew.-%, besonders bevorzugt 5 bis 50 Gew.-% und darüber hinaus bevorzugt 10 bis 25 Gew.-% an β-Hydroxypropionsäure, jeweils bezogen auf das Gesamtgewicht der wässrigen Lösung, in den Reaktor eingebracht. Die Druck- und Temperaturbedingungen im Inneren des Reaktors werden so gewählt, dass die β-Hydroxypropionsäure bzw. die wässrigen Lösung bei ihrem Eintritt in der Reaktor in gasförmiger Form vorliegt.

Die Dehydratisierung in der Gasphase erfolgt vorzugsweise im Temperaturbereich zwischen 200 und 400°C, besonders bevorzugt zwischen 250 und 350°C. Der Druck im Inneren des Reaktors liegt bei der Gasphasendehydratisierung vorzugsweise in einem Bereich von 0,1 bis 50 bar, besonders bevorzugt in einem Bereich von 0,2 bis 10 bar und am meisten bevorzugt in einem Bereich von 0,5 bis 5 bar.

Die Menge an in den Reaktor eingebrachter β-Hydroxypropionsäure liegt bei der Gasphasendehydratisierung vorzugsweise in einem Bereich von 10 bis 100 Vol-%, besonders bevorzugt in einem Bereich von 20 bis 100 Vol-% und am meisten bevorzugt in einem Bereich von 30 bis 100 Vol-%.

Gemäß einer anderen besonderen Ausführungsform des erfindungsgemäßen Verfahrens wird die Dehydratisierung in der Flüssigphase durchgeführt. Die Flüssigphasendehydratisierung kann ebenfalls in allen dem Fachmann bekannten Apparaturen durchgeführt werden, in denen ein Fluid auf eine gewünschte Reaktionstemperatur erhitzt werden kann, wobei die Apparatur mit einem Druck beaufschlagt werden kann, der ausreicht, um die Reaktionskomponenten unter den gewünschten Temperaturbedingungen flüssig zu halten.

Gemäß einer besonderen Ausführungsform des erfindungsgemäßen Verfahrens umfasst das Verfahren der Flüssigphasendehydratisierung einen ersten Verfahrensschritt, bei dem reine β-Hydroxypropionsäure oder eine wässrige Lösung beinhaltend 5 bis 100 Gew.-%, besonders bevorzugt 20 bis 100 Gew.-% und am meisten bevorzugt 50 bis 100 Gew.-% an β-Hydroxypropionsäure, bezogen auf das Gesamtgewicht der wässrigen Lösung, in einen Reaktor eingebracht wird. Die Druck- und Temperaturbedingungen im Inneren des Reaktors werden so gewählt, dass die β-Hydroxypropionsäure bzw. die wässrigen Lösung bei ihrem Eintritt in der Reaktor in flüssiger Form vorliegt. Gemäß einer besonderen Ausführungsform des erfindungsgemäßen Verfahrens, bei dem eine Dehydratisierung in der Flüssigphase durchgeführt wird, wird die β-Hydroxypropionsäure bzw. die wässrige Lösung im inneren des Dehydratisierungsreaktors derart über ein Katalysatorfestbett geführt, dass die flüssige Phase über die Oberfläche der Katalysatorpartikel rieselt. Eine derartige Vorgehensweise kann beispielsweise in einem Rieselbettreaktor durchgeführt werden.

Die Dehydratisierung in der Flüssigphase erfolgt vorzugsweise im Temperaturbereich zwischen 200 und 350°C, besonders bevorzugt zwischen 250 und 300°C. Der Druck im Inneren des Reaktors liegt bei der Flüssigphasendehydratisierung vorzugsweise in einem Bereich von 1 bis 50 bar, besonders bevorzugt in einem Bereich von 2 bis 25 bar und am meisten bevorzugt in einem Bereich von 3 bis 10 bar.

Die Katalyse der Dehydratisierung kann sowohl bei der Gasphasendehydratisierung als auch bei der Flüssigphasendehydratisierung homogen oder heterogen erfolgen.

Bei der homogenen Katalyse wird der Katalysator, bei dem es sich dann vorzugsweise um eine anorganische Säure wie etwa Phosphorsäure oder Schwefelsäure handelt, zunächst mit der reinen β-Hydroxypropionsäure oder mit der wässrigen Lösung beinhaltend die β-Hydroxypropionsäure in Kontakt gebracht. Anschließend wird die so erhaltene Zusammensetzung in den Reaktor eingebracht und unter den gewünschten Druck- und Temperaturbedingungen in Acrylsäure überführt. Denkbar ist auch, die anorganische Säure unabhängig von der β-Hydroxypropionsäure bzw. der wässrigen Lösung in den Reaktor einzubringen. In diesem Fall weist der Reaktor mindestens zwei Zuleitungen, eine für die β-Hydroxypropionsäure bzw. die wässrigen Lösung beinhaltend die β-Hydroxypropionsäure und eine für den Katalysator, auf. Wird die Dehydratisierung in flüssiger Phase in einem Rieselbettreaktor durchgeführt, so ist es bevorzugt, dass der Katalysator zusammen mit der β-Hydroxypropionsäure bzw. der wässrigen Lösung beinhaltend die β-Hydroxypropionsäure im Kopfbereich des Reaktors eingebracht wird.

Bei der heterogenen Katalyse befindet sich der Katalysator in Form eines festen Substrates im Reaktionsraum, beispielsweise in Form einer Festbettschüttung, in Form von mit Katalysator beschichteten Platten, vorzugsweise Thermoblechen, welche im Inneren des Reaktors angeordnet sind oder aber in Form von mit Katalysator beschichteten Reaktorwänden. Mögliche Reaktoren sind beispielsweise in der DE-A-198 48 208, DE-A-100 19 381 und EP-A-1 234 612 beschrieben. Im Falle der heterogenen Katalyse sind als Katalysatoren mit anorganischen Säure in Kontakt gebrachte, vorzugsweise imprägnierte porenförmiger Trägerkörper bevorzugt. Die β-Hydroxypropionsäure bzw. die wässrige Lösung beinhaltend die β-Hydroxypropionsäure wird dann in dampfförmiger oder flüssiger Form mit der Oberfläche des festen Katalysatormaterials in Kontakt gebracht.

Als Reaktionsgemisch, welches im Anschluss an die Dehydratisierung erhalten wird, wird entweder eine wässrige Acrylsäurelösung, die keine Katalysatorbestandteile enthält (eine solche wird im Falle einer heterogen katalysierten Dehydratisierung erhalten) oder aber eine wässrige Acrylsäurelösung, welche Katalysatoren beinhaltet (eine solche wird im Falle einer homogen katalysierten Dehydratisierung erhalten) erhalten. Weiterhin kann die wässrige Acrylsäurelösung in flüssiger Form (sofern die Dehydratisierung in der Flüssigphase erfolgt ist) oder gasförmig (sofern die Dehydratisierung in der Gasphase erfolgt ist) vorliegen.

Diese wässrige Acrylsäuerlösung kann grundsätzlich durch jedes dem Fachmann bekanntes Aufreinigungsverfahren aufgereinigt werden, welches herkömmlicherweise zur Aufreinigung verunreinigter, durch katalytische Gasphasenoxidation von Propylen erhaltener Acrylsäure Anwendung findet.

Wurde die Dehydratisierung in der Gasphase durchgeführt, so ist es bevorzugt, dass die Acrylsäure zunächst unter Erhalt einer wässrigen Acrylsäurelösung kondensiert wird. Dabei kann grundsätzlich jedes dem Fachmann bekannte Kondensationsverfahren eingesetzt werden, beispielsweise eine fraktionierte Kondensation, wie sie in WO-A-2004/035514, WO-A-03/014172 oder EP-A-EP 1 163 201 beschrieben ist, oder durch eine Totalkondensation, wie sie in der EP-A-0 695 736 beschrieben ist. Denkbar ist auch, bei der Kondensation zusätzliches Lösungsmittel, insbesondere Wasser, zuzusetzen, um die Acrylsäure möglichst vollständig zu absorbieren.

Die nach der Kondensation erhaltene, wässrige Acrylsäurelösung oder aber die im Falle der Flüssigphasendehydratisierung erhaltene wässrige Acrylsäurelösung kann dann in weiteren Aufreinigungsschritten von Wasser und anderen Verunreinigungen befreit werden. Dabei kann zunächst das Wasser in Gegenwart eine Schleppmittels durch Azeotropdestillation entfernt werden, wie dies beispielsweise in der DE-A-198 53 064 beschrieben ist. Denkbar ist auch der Einsatz hochsiedender organischer Lösungsmittel zur Absorption der Acrylsäure, wie dies beispielsweise in der EP-A-0 974 574 offenbart ist. Neben diesen Destillativen Verfahren können auch Membranen zur Entwässerung eingesetzt werden, wie dies etwa in DE-A-44 01 405 vorgeschlagen wird. Denkbar ist weiterhin eine Aufreinigung der im Falle der Flüssigphasendehydratisierung gewonnen oder durch Kondensation erhaltenen wässrigen Acrylsäurelösung durch Kristallisationsverfahren, wobei gegebenenfalls der aufzureinigenden wässrigen Acrylsäurelösung vor der Kristallisation ein Trennmittel, beispielsweise Toluol, zugesetzt werden kann.

Die nach dem Entwässern erhaltene Acrylsäure kann in weiteren Verfahrensschritten noch weiter aufgereinigt werden. So können noch enthaltene, hochsiedende Verunreinigungen durch weitere Destillationsschritte entfernt werden. Besonders bevorzugt ist es jedoch, wenn die nach dem Entwässern erhaltene Acrylsäure durch Kristallisationsverfahren weiter aufgereinigt wird, wie dies etwa in DE-A-101 49 353 beschrieben ist. Vor der Kristallisation kann die aufzureinigende Acrylsäure auch mit einem Trennmittel, vorzugsweise mit Toluol, in Kontakt gebracht werden.

Nach der Aufreinigung der bei der Dehydratisierung anfallenden Acrylsäurehaltigen Zusammensetzung wird vorzugsweise eine Acrylsäure erhalten, die zu mindestens 99 Gew.-%, besonders bevorzugt zu mindestens 99,5 Gew.-% und am meisten bevorzugt zu mindestens 99,9 Gew.-% auf Acrylsäure basiert.

Gemäß einer Ausführungsform des erfindungsgemäßen Verfahrens zur Herstellung von Polymeren durch radikalische Polymerisation von Acrylsäure umfasst das Syntheseverfahren der Acrylsäure folgende Verfahrenschritte:
- Spaltung eines Kohlenhydrates mittels eines Enzyms oder mindestens eines Bestandteils von einem Enzym unter Bildung von β-Hydroxypropionsäure, vorzugsweise in einem Fermenter;
- Isolierung der β-Hydroxypropionsäure aus der Fermentationsbrühe unter Erhalt einer wässrigen β-Hydroxypropionsäure-Lösung, wobei die Isolierung gegebenenfalls durch in Kontakt bringen der von den Mikroorganismen befreiten Fermentationslösung mit einem Amin, Extraktion des so erhaltenen β-Hydroxypropionsäure-Salzes und destillative Aufreinigung des Extraktes erhalten werden kann;
- heterogene Dehydratisierung der β-Hydroxypropionsäure in der Gasphase oder in der Flüssigphase;
- Kondensation des gasförmigen Reaktionsgemisches unter Erhalt einer wässrigen Acrylsäurelösung;
- Gegebenenfalls Zugabe eines Trennmittels, vorzugsweise Toluol, zu der wässrigen Acrylsäurelösung und Abtrennung der Acrylsäure mittels Kristallisation.

Gemäß einer anderen Ausführungsform des erfindungsgemäßen Verfahrens zur Herstellung von Polymeren durch radikalische Polymerisation von Acrylsäure umfasst das Syntheseverfahren der Acrylsäure folgende Verfahrenschritte:
- Spaltung eines Kohlenhydrates mittels eines Enzyms oder mindestens eines Bestandteils von einem Enzymen unter Bildung von β-Hydroxypropionsäure, vorzugsweise in einem Fermenter;
- Isolierung der β-Hydroxypropionsäure aus der Fermentationsbrühe unter Erhalt einer reinen β-Hydroxypropionsäure, wobei die Isolierung gegebenenfalls durch in Kontakt bringen der von den Mikroorganismen befreiten Fermentationslösung mit einem Amin, Extraktion des so erhaltenen β-Hydroxypropionsäure-Salzes und destillative Aufreinigung des Extraktes erhalten werden kann;
- heterogene Dehydratisierung der β-Hydroxypropionsäure in der Gasphase oder in der Flüssigphase;
- Kondensation des gasförmigen Reaktionsgemisches unter Erhalt einer wässrigen Acrylsäurelösung;
- Gegebenenfalls Zugabe eines Trennmittels, vorzugsweise Toluol, zu der wässrigen Acrylsäurelösung und Abtrennung der Acrylsäure mittels Kristallisation.

Gemäß einer besonderen Ausführungsform des erfindungsgemäßen Verfahrens zur Herstellung von Polymeren durch radikalische Polymerisation von Acrylsäure wurden mindestens 80 Gew.-%, vorzugsweise mindestens 90 Gew.-% und am meisten bevorzugt mindestens 95 Gew.-% der zur Herstellung der Polymere eingesetzten Acrylsäuremonomere durch das vorstehend beschriebene Syntheseverhalten umfassend den Verfahrensschritt des Spaltens eines organischen Materials mittels eines Enzyms oder mindestens eines Bestandteils von einem Enzym erhalten.

Nachdem die Acrylsäure gemäß den vorstehend beschriebenen Aufreinigungsverfahren aus den nach der Dehydratsierung erhaltenen Zusammensetzungen aufgereinigt wurde, erfolgt die radikalische Polymerisation der Acrylsäuremonomere durch den Fachmann bekannte Polymerisationsverfahren. Wenn es sich bei den Polymeren um vernetzte, teilneutralisierte Polyacrylate handelt, so wird hinsichtlich der genauen Vorgehensweise auf das 3. Kapitel (Seite 69ff) in "Modern Superabsorbent Polymer Technology", F. L. Buchholz und A. T. Graham (Herausgeber) in, Wiley-VCH, New York, 1998 verwiesen.

Einen Beitrag zur Lösung der eingangs genannten Aufgaben leisten auch wasserabsorbierende Polymergebilde, erhältlich durch radikalische Polymerisation der durch das vorstehend beschriebene Syntheseverfahren erhältlichen Acrylsäure in Gegenwart von Vernetzern.

Einen Beitrag zur Lösung der eingangs genannten Aufgaben leisten auch wasserabsorbierende Polymergebilde, welche zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure basieren, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch ein Syntheseverfahren erhalten wurden, welches den Verfahrensschritt der Spaltung eines organischen Materials mittels eines Enzyms oder mindestens eines Bestandteils von einem Enzym umfasst.

Gemäß einer besonderen Ausführungsform der erfindungsgemäßen wasserabsorbierende Polymergebilde basieren diese zu mindestens 25 Gew.-%, vorzugsweise zu mindestens 35 Gew.-% und am meisten bevorzugt zu mindestens 45 Gew.-% auf natürlichen, biologisch abbaubaren Polymeren, vorzugsweise auf Kohlehydraten wie etwa Zellulose oder Stärke.

Weiterhin ist es erfindungsgemäß bevorzugt, dass die wasserabsorbierenden Polymergebilde mindestens ein der folgenden Eigenschaften aufweisen:
- einen gemäß ERT 441.2-02 (ERT = Edana Recommended Test Method) bestimmten CRC-Wert (CRC = Centrifugation Retention Capacity) von mindestens 20 g/g, vorzugsweise mindestens 25 g/g und am meisten bevorzugt mindestens 30 g/g, wobei ein CRC-Wert von 60 g/g, vorzugsweise von 50 g/g nicht überschritten wird;
- eine gemäß ERT 442.2-02 bestimmte Absorption unter einem Druck von 20 g/cm² von mindestens 16 g/g, vorzugsweise mindestens 18 g/g und am meisten bevorzugt mindestens 20 g/g, wobei ein Wert von 50 g/g, vorzugsweise von 40 g/g nicht überschritten wird.

Einen weiteren Beitrag zur Lösung der eingangs genannten Aufgaben leisten wasserabsorbierende Polymergebilde, welche durch folgende Eigenschaften gekennzeichnet sind:
(β1) das Polymergebilde basiert zu mindestens 25 Gew.-%, vorzugsweise zu mindestens 50 Gew.-%, noch mehr bevorzugt zu mindestens 75 Gew.-% und am meisten bevorzugt zu mindestens 95 Gew.-%, auf Acrylsäure, wobei mindestens 80 Gew.-%, vorzugsweise mindestens 90 Gew.-% und am meisten bevorzugt mindestens 95 Gew.-% der zur Herstellung der wasserabsorbierenden Polymergebilde eingesetzten Acrylsäuremonomere durch ein Syntheseverfahren erhalten wurden, welches den Verfahrensschritt der Spaltung eines organischen Materials mittels eines Enzyms oder mindestens eines Bestandteils von einem Enzym umfasst.
(β2) das Polymergebilde weist eine gemäß dem modifizierten Sturm-Test gemäß Anhang V zur Richtlinie 67/548/EWG bestimmte biologische Abbaubarkeit nach 28 Tagen von mindestens 25 %, vorzugsweise mindestens 35 % und am meisten bevorzugt mindestens 45 % auf, wobei ein Wert von maximal 75 bis 95 Gew.-% als Obergrenze im allgemeinen nicht überschritten wird;
(β3) das Polymergebilde weist einen gemäß ERT 441.2-02 bestimmten CRC-Wert von mindestens 20 g/g, vorzugsweise mindestens 25 g/g und am meisten bevorzugt mindestens 30 g/g auf, wobei ein CRC-Wert von maximal 60 g/g als Obergrenze im allgemeinen nicht überschritten wird.

In einer weiteren Ausgestaltung des im vorstehenden Abschnitt beschriebenen Polymergebildes weist dieses mindestens die Eigenschaften ß1 und ß2 auf. Alle in diesem Text für die Polymergebilde angegebenen Weiterbildungen gelten auch für das Polymergebilde dieses Absatzes.

Einen andern Beitrag zur Lösung der eingangs genannten Aufgaben leisten wasserabsorbierende Polymergebilde, welches zu mindestens 10, vorzugsweise mindestens 25, besonders bevorzugt mindestens 50 und darüber hinaus bevorzugt mindestens 75 sowie ferner bevorzugt mindestens 80 Gew.-%, jeweils bezogen auf das Polymergebilde, auf Acrylsäure basiert, welche durch folgende Eigenschaften gekennzeichnet sind:
(ε1) das Polymergebilde zeigt einen Nachhaltigkeitsfaktor von mindestens 10, vorzugsweise mindestens 20, besonders bevorzugt mindestens 50, darüber hinaus bevorzugt mindestens 75, ferner bevorzugt mindestens 85 und weiterhin bevorzugt mindestens 95;
(s2) das Polymergebilde weist eine gemäß dem modifizierten Sturm-Test gemäß Anhang V zur Richtlinie 67/548/EWG bestimmte biologische Abbaubarkeit nach 28 Tagen von mindestens 25 %, vorzugsweise mindestens 35 % und am meisten bevorzugt mindestens 45 % auf, wobei ein Wert von maximal 75 bis 95 % als Obergrenze im allgemeinen nicht überschritten wird;
(ε3) das Polymergebilde weist einen gemäß ERT 441.2-02 bestimmten CRC-Wert von mindestens 20 g/g, vorzugsweise mindestens 25 g/g und am meisten bevorzugt mindestens 29 g/g auf, wobei ein CRC-Wert von 60 g/g, als Obergrenze im allgemeinen nicht überschritten wird.

In einer anderen Ausgestaltung des im vorstehenden Abschnitt beschriebenen Polymergebildes weist dieses mindestens die Eigenschaften ε1 und ε2 auf. Alle in diesem Text für die Polymergebilde angegebenen Weiterbildungen gelten auch für das Polymergebilde dieses Absatzes.

In manchen Fällen können die vorstehend genannten Obergrenzen auch bis zu 10 % oder auch bis zu 20 % niedriger liegen. Bei den in den beiden vorstehenden Absätzen beschriebenen Polymergebilden ist es bevorzugt, dass diese neben der Acrylsäure weiterhin auf einem Zwei- oder Mehrfachzucker basieren. Diese Zwei- oder Mehrfachzucker liegen vorzugsweise in einer Menge von mindestens 1 Gew.-%, vorzugsweise mindestens 5 Gew.-% und darüber hinaus bevorzugt mindestens 15 Gew.-%, jeweils bezogen auf das Polymergebilde, als ein weitere Bestandteil des Polymergebildes vor, so dass die Summe der Gew.-% der Bestandteile des wasserabsorbierenden Polymergebildes 100 Gew.-% ergibt. Bei derartigen Zuckern sind Kettenmehrfachzucker bevorzugt, die vorzugsweise ein mittels Gelpermeationschromatographie und Lichtstreuung ermitteltes Zahlenmittel des Molekulargewichts im Bereich von 10.000 bis 1.000.000 und vorzugsweise im Bereich von 50.000 bis 500.000 g/mol aufweisen. Diese bestehen vorzugsweise aus linearen und damit unverzeigten Ketten. Als derartige Zucker kommen alle dem Fachmann bekannten und als geeignet erscheinenden Zuckerverbindungen in betracht. So sind beispielsweise Zellulose und Stärke zu nennen, wobei ein oder mindestens zwei verschiedenen Stärken bevorzugt sind. Unter den Stärken sind wiederum amylosehaltige Stärken bevorzugt. Der Amylosegehalt liegt vorzugsweise in einem Bereich von 10 bis 80 und besonders bevorzugt in einem Bereich von 20 bis 70 Gew.-%, jeweils bezogen auf die Stärke. Weiterhin ist es bevorzugt, dass die Zwei- oder Mehrfachzucker eine Teilchengröße aufweisen, bei der mindestens 50 Gew.-%, vorzugsweise mindestens 70 Gew.-% und darüber hinaus bevorzugt mindestens 85 Gew.-% der Teilchen kleiner 50)µm sind. Die Teilchengröße wird durch Siebanalyse bestimmt. Derartige Produkte sind beispielsweise unter der Marke Eurylon^{®} 7 oder Foralys^{®} 380 der Firma Roquette - Frankreich - kommerziell erhältlich.

Hergestellt werden können und damit erhältlich sind derartige wasserabsorbierende Polymergebilde vorzugsweise durch
- Bereistellen eines oberflächenvernetzten wasserabsorbierenden Polymers;
- Mischen des oberflächenvernetzten wasserabsorbierenden Polymergebildes mit einem Zwei- oder Mehrfachzucker.

Hierbei ist es bevorzugt, dass das wasserabsorbierende Polymer zu mindesten 50 Gew.-%, vorzugsweise mindestens 80 Gew.-% und darüber hinaus bevorzugt mindestens 95 Gew.-% auf Acrylsäure basiert, die aus dem erfindungsgemäßen Dehydratisierungsverfahren stammt und teilneutralisiert mit einem Vernetzer zur Polymerisation eingesetzt wird. Die Oberflächenvernetzung wird durch die hier bereits beschriebene Nachvernetzung erreicht.

Der Nachhaltigkeitsfaktor gibt an, zu welchem Anteil das Polymergebilde auf Stoffen basierend auf nichtfossilen, nachwachsendem organischen Material basiert. Bei einem Nachhaltigkeitsfaktor von 100 besteht das Polymergebilde vollständig aus auf nichtfossilen, nachwachsendem organischen Materialen basierenden Stoffen.

Einen weiteren Beitrag zur Lösung der eingangs beschriebenen Aufgaben liefert ein Verbund, beinhaltend die erfindungsgemäßen wasserabsorbierenden Polymergebilde bzw. wasserabsorbierenden Polymergebilde, die durch radikalische Polymerisation der durch das vorstehend beschriebene Syntheseverfahren erhältlichen Acrylsäure in Gegenwart von Vernetzern erhältlich sind. Es ist dabei bevorzugt, dass die erfindungsgemäßen Polymergebilde und das Substrat miteinander fest verbunden sind. Als Substrate sind Folien aus Polymeren, wie beispielsweise aus Polymethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass die Polymergebilde in einer Menge von mindestens 50 Gew.-%, vorzugsweise mindestens 70 Gew.-% und noch mehr bevorzugt mindestens 90 Gew.-%, bezogen auf das Gesamtgewicht aus Polymergebilde und Substrat, in dem Verbund enthalten sind.

In einer besonders bevorzugten Ausführungsform des erfindungsgemäßen Verbundes handelt es sich um einen flächenförmigen Verbund, wie er in der WO-A-02/056812 als "absorbent material" beschrieben ist. Der Offenbarungsgehalt der WO-A-02/056812, insbesondere hinsichtlich des genauen Aufbaus des Verbundes, des Flächengewichtes seiner Bestandteile sowie seiner Dicke wird hiermit als Referenz eingeführt und stellt einen Teil der Offenbarung der vorliegenden Erfindung dar.

Einen weiteren Beitrag zur Lösung der eingangs genannten Aufgaben liefert ein Verfahren zur Herstellung eines Verbundes, wobei die erfindungsgemäßen wasserabsorbierenden Polymergebilde bzw. die wasserabsorbierenden Polymere, die durch radikalische Polymerisation der durch das vorstehend beschriebene Syntheseverfahren erhältlichen Acrylsäure in Gegenwart von Vernetzern erhältlich sind, und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der eingangs genannten Aufgaben liefert auch ein Verbund erhältlich nach dem vorstehend beschriebenen Verfahren.

Einen weiteren Beitrag zur Lösung der eingangs genannten Aufgaben liefern chemische Produkte beinhaltend die erfindungsgemäßen wasserabsorbierenden Polymergebilde oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen wasserabsorbierenden Polymergebildes oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Einen besonderen Beitrag zur Lösung der eingangs genannten Aufgaben leistet auch die Verwendung von Acrylsäure, erhalten durch ein Syntheseverfahren, welches folgenden Verfahrensschritt umfasst:
- Spaltung eines organischen Materials mittels Enzymen oder mindestens eines Bestandteils von Enzym,
zur Herstellung wasserabsorbierender Polymergebilde.

Einen Beitrag zur Lösung der eingangs genannten Aufgaben leistet auch eine Vorrichtung zur Herstellung von Acrylsäure, umfassend die folgenden, fluidleitend miteinander verbunden Vorrichtungsbestandteile:
(γ1) mindestens einen Bioreaktor umfassend
   (γ1_1) einen Reaktionsraum,
   (γ1_2) eine Zuführung für ein organisches Material,
   (γ1_3) eine Zuführung für Nährstoffe oder Nährstofflösungen,
   (γ1_4) eine Ableitung für ein β-Hydroxypropionsäure beinhaltendes Reaktionsgemisch
   (γ1_5) mindestens eine Rührvorrichtung,
   (γ1_6) mindestens eine Heizvorrichtung,
   (γ1_7) gegebenenfalls mindestens eine Begasungseinheit, sowie
   (γ1_8) gegebenenfalls mindestens eine Messsonde;
(γ2) eine mit der Ableitung (γ1_4) des Biorealetors (γ1) fluidleitend verbundene erste Aufreinigungseinheit zur Aufreinigung von β-Hydroxypropionsäure;
(γ3) eine mit der Aufreinigungseinheit (γ2) verbunden Dehydratisierungsreaktor, umfassend
   (γ3_1) einen Reaktionsraum, aufweisend einen Katalysator
   (γ3_2) eine Zuführung für aufgereinigte β-Hydroxypropionsäure,
   (γ3_3) eine Ableitung für ein Acrylsäure beinhaltendes Reaktionsgemisch, sowie
   (γ3_4) mindestens eine Heizvorrichtung,
(γ4) gegebenenfalls eine mit der Ableitung (γ3_3) verbundene zweite Aufreinigungseinheit zur Aufreinigung von Acrylsäure.

Unter "fluidleitend" wird erfindungsgemäß verstanden, dass Gase oder Flüssigkeiten, Suspensionen eingeschlossen, oder deren Mischungen durch entsprechende Leitungen geführt werden. Hierzu lassen sich insbesondere Rohleitungen, Pumpen und der gleichen einsetzen.

Als Bioreaktoren (γ1) können alle dem Fachmann bekannten Reaktortypen eingesetzt werden, in denen organisches Material unter der Einwirkung von Mikroorganismen zersetzt werden kann. Sofern in dem Reaktor ein Fermentationsprozess unter Beteilung von Mikroorganismen zur Spaltung organischen Materials durchgeführt wird, ist es bevorzugt, wenn der Reaktor neben einer geeigneten Rührvorrichtung auch Messsonden, vorzugsweise zur Bestimmung des pH-Wertes, der CO₂-Konzentration sowie der Temperatur aufweist. Weiterhin kann der Bioreaktor auch ein Trägermaterial aufweisen, auf dessen Oberfläche die Mikroorganismen immobilisiert sind. Als Trägermaterialen für die Immobilisierung von Mikroorganismen kommen insbesondere poröse Steine, beispielsweise Lava oder Blähton sowie Kohle und verschiedene Arten von Kunststoffkörpern in Betracht. Die Korngröße bewegt sich dabei je nach Werkstoff und Größe des Bioreaktors vorzugsweise zwischen etwa 5 und 50 mm.

Als Bioreaktoren können jedoch auch Reaktoren eingesetzt werden, welche ein Trägermaterial aufweisen, an dessen Oberfläche Enzyme, welche die Spaltung eines organischen Materials unter Bildung von β-Hydroxypropionsäure ermöglichen, immobilisiert sind.

Bei der ersten Aufreinigungseinheit für β-Hydroxypropionsäure (γ2) kann es sich beispielsweise um eine mit einem Ultrafilter als Vorfilter versehene mehrstufige Trennanlage handeln, wie sie in der DE-A-197 18 608 beschrieben ist. Bei dem Dehydratisierungsreakor (γ3) handelt es sich vorzugsweise um einen Rohrbündelreaktor mit oder ohne Katalysatorschüttung oder aber um einen Rieselbettreaktor.

Bei der zweiten Aufreinigungseinheit (γ4) kann es sich um jede dem Fachmann bekannte Aufreinigungseinheit handeln, die zur Aufreinigung von durch Gasphasenoxidation von Propylen erhaltenen Acrylsäure eingesetzt wird. Diese Aufreinigungseinheiten umfassen vorzugsweise Destillationsvorrichtungen und/oder Kristallisationsvorrichtungen.

Einen Beitrag zur Lösung der eingangs genannten Aufgaben liefert auch ein Verfahren zur Herstellung von Acrylsäure aus Kohlenhydraten, wobei die vorstehend beschriebene Vorrichtung eingesetzt wird. Schließlich leistet auch die durch dieses Verfahren erhältliche Acrylsäure einen Beitrag zur Lösung der eingangs genannten Aufgaben. Dies Acrylsäure ist vorzugsweise durch mindestens eine, vorzugsweise alle der folgenden Eigenschaften gekennzeichnet:
- einen Gehalt an Aldehyden, insbesondere an Benzaldehyd, von weniger als 5 ppm, besonders bevorzugt weniger als 1 ppm, darüber hinaus bevorzugt weniger als 0,1 ppm und am meisten bevorzugt weniger als 0,01 ppm;
- einen Gehalt an Maleinsäure oder Maleinsäureanliydrid von weniger als 5 ppm, besonders bevorzugt weniger als 1 ppm, darüber hinaus bevorzugt weniger als 0,1 ppm und am meisten bevorzugt weniger als 0,01 ppm;
- einen Gehalt an Essigsäure von weniger als 5 ppm, besonders bevorzugt weniger als 1 ppm, darüber hinaus bevorzugt weniger als 0,1 ppm und am meisten bevorzugt weniger als 0,01 ppm;
- einen Gehalt an Ketonen, insbesondere an PTA, von weniger als 5 ppm, besonders bevorzugt weniger als 1 ppm, darüber hinaus bevorzugt weniger als 0,1 ppm und am meisten bevorzugt weniger als 0,01 ppm.

Die Erfindung wird nun anhand von nicht limitierenden Zeichnungen und Beispielen näher erläutert.

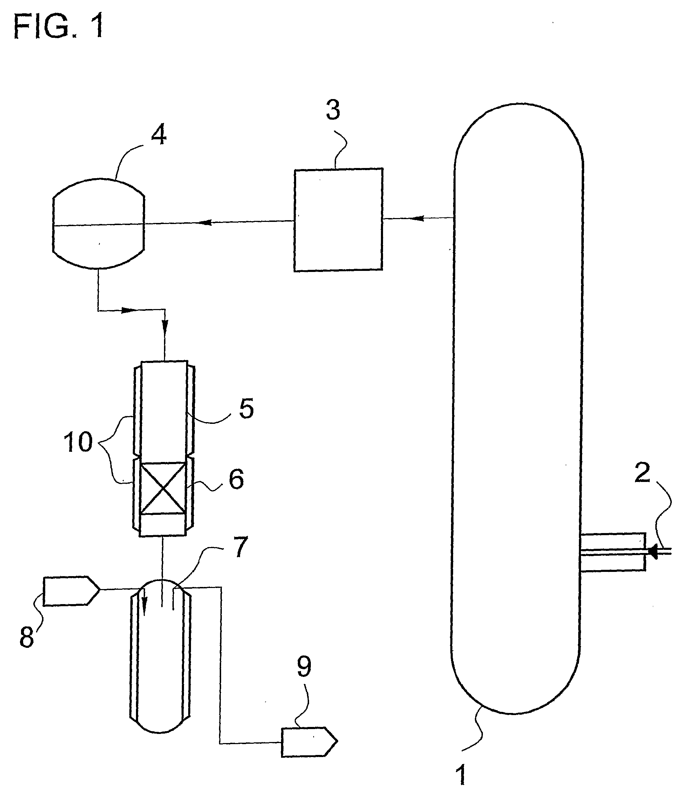

Es zeigt die Figur 1 eine erfindungsgemäße Vorrichtung zur Herstellung von Acrylsäure, bei der eine Dehydratisierung in der Gasphase erfolgt.

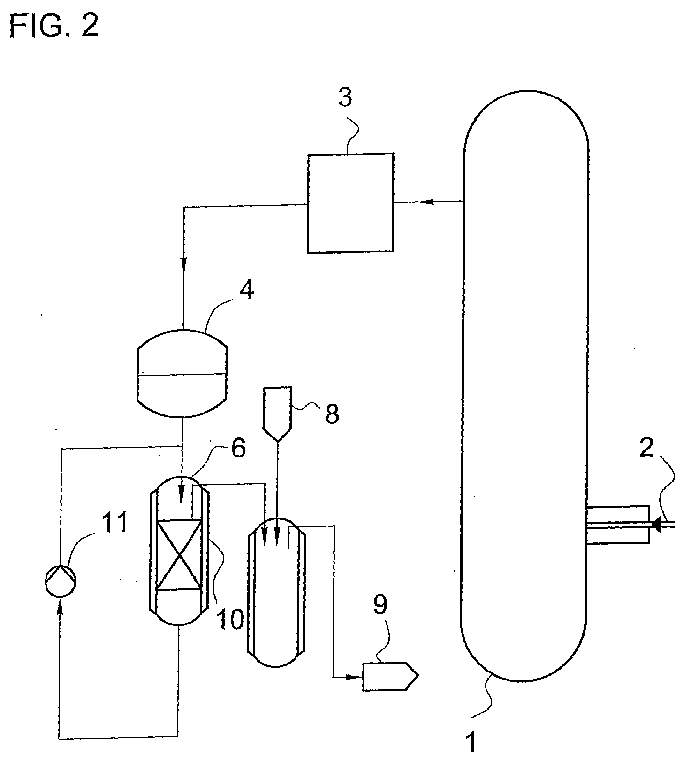

Es zeigt die Figur 2 eine erfindungsgemäße Vorrichtung zur Herstellung von Acrylsäure, bei der eine Dehydratisierung in der Flüssigphase erfolgt.

Gemäß Figur 1 wird über eine Zuführung 2 das zu spaltende organische Material, bei dem es sich vorzugsweise um Kohlenhydrate, besonders bevorzugt um Glukose, handelt, in einen Bioreaktor 1 eingebracht, in dem sich ein geeignetes Nährmedium befindet. Weiterhin können in dem Bioreaktor Mikroorganismen im Nährmedium suspendiert oder auf einem Substrat immobilisiert vorliegen. Denkbar ist auch, dass Enzyme, welche die Spaltung des organischen Materials in β-Hydroxypropionsäure katalysieren, auf einem geeigneten Substrat immobilisiert sind. Nach der Spaltung des organischen Materials wird die Fermentationsbrühe in einer ersten Aufreinigungsvorrichtung 3 gereinigt. Vorzugsweise umfasst diese Aufreinigungsvorrichtung 3 einen Ultrafilter mit einer Ausschlussgröße in einem Bereich von 20 bis 200 kDa als Vorfilter zur Abtrennung der Zellen sowie weitere Vorrichtungsbestandteile wie etwa Elektrodialysevorrichtungen, mit denen eine Aufreinigung der β-Hydroxypropionsäure erfolgt. Die Aufgereinigte β-Hydroxypropionsäure wird entweder in reiner Form oder in Form einer wässrigen Lösung in einen Behälter 4 als Eduktvorlage überführt. Von hier aus gelangt die β-Hydroxypropionsäure bzw. die wässrige Lösung beinhaltend die β-Hydroxypropionsäure in einen Verdampfer 5, welcher eine Heizvorrichtung 10 umfasst, in dem die einzelnen Komponenten der Zusammensetzung verdampft werden. Die so erhaltene Gasphase wird dann mit einem Katalysatorbett 6 in Kontakt gebracht, welches vorzugsweise auf einem mit Phosphorsäure imprägniertem, porenförmigen Material basiert und ebenfalls mittels einer Heizvorrichtung 10 auf die gewünschte Dehydratisierungstemperatur erhitzt werden kann. Die nach dem passieren des Katalysatorbettes erhaltene gasförmige Zusammensetzung beinhaltend Wasser und Acrylsäure wird in einer Kondensationsvorrichtung 7 unter Erhalt einer wässrigen Acrylsäurelösung kondensiert. Gegebenenfalls kann über eine Zuleitung 8 zusätzliches Lösungsmittel, wie etwa Wasser, zur Absorption der Acrylsäure zugesetzt werden. Über eine Ableitung 9 kann die wässrige Acrylsäurelösung weiteren Reinigungsvorrichtungen, insbesondere Kristallisations- und/oder Destillationsvorrichtungen zugeführt werden, um eine möglichst reine Acrylsäure zu erhalten.

Die Herstellung von Acrylsäure, bei der eine Flüssigphasendehydratisierung erfolgt (Fig. 2) erfolgt im Wesentlichen ebenso wie die in der Figur 1 dargestellte Gasphasendehydratisierung. Jedoch wird die nach der Aufreinigung der Fermentationslösung erhaltene β-Hydroxypropionsäure bzw. die wässrige Lösung beinhaltend die β-Hydroxypropionsäure nicht mittels einen Verdampfers verdampft, sondern in flüssiger Form in den Reaktor zur Dehydratisierung überführt. Um eine möglichst vollständige Dehydratisierung zu erreichen, kann es vorteilhaft sein, die wässrige β-Hydroxypropionsäure-Lösung mittels einer Pumpe 11 im Kreislauf zu führen, wie dies in der Fig. 2 gezeigt ist.

### BEISPIEL 1

### (Vergleichsbeispiel)

β-Hydroxypropionsäure wurde fermentativ gemäß dem Beispiel 8 der WO-A-0242418 hergestellt. Aus der von Zellen befreiten Fermentationslösung wurde die β-Hydroxypropionsäure gemäß dem im Beispiel 2 der WO-A-02/090312 aus der Zusammensetzung abgetrennt. Die auf diese Weise gewonnene reine β-Hydroxypropionsäure wird in einer Gasphasendehydratisierung in Acrylsäure überführt. Als Katalysatormaterial werden 100 g Rosenthal-Kugeln (alpha-Al₂O₃) mit einem Durchmesser von 3 mm eingesetzt, die zuvor mit 25 g einer 20 gew.-%igen Phosphorsäure 1 Stunde gemischt wurden. Am Rotationsverdampfer wird bei 80 °C das überschüssige Wasser abgezogen. 100 ml dieses Katalysators (-5,6 <Ho <-3) werden in einem Stahlrohr von 15 mm Durchmesser eingefüllt.

Die nach der Dehydratisierung der β-Hydroxypropionsäure erhaltene wässrige Acrylsäurelösung wird durch Azeotropdestillation gemäß der Lehre der DE-A-198 53 064 aufgereinigt. Die auf diese Weise erhaltene Acrylsäure enthielt keine nachweisbaren Mengen an PTA oder Benzaldehyd.

Eine Monomerenlösung bestehend aus 280 g der vorstehend gewonnenen Acrylsäure, die zu 70 Mol-% mit Natronlauge neutralisiert wurde, 466,8 g Wasser, 1,4 g Polyethylenglykol-300-diacrylat und 1,68 g Allyloxypolyethylenglykolacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,1 g 2,2'-Azobis-2-amidinpropan-dihydrochlorid in 10 g H₂O, 0,3 g Natriumperoxydisulfat in 10 g H₂O, 0,07 g 30%ge Wasserstoffperoxidlösung in 1 g H₂O und 0,015 g Ascorbinsäure in 2 g H₂O zugesetzt. Nachdem die Endtemperatur von ca. 100°C erreicht war, wurde das entstandene Gel zerkleinert und bei 150°C 90 Minuten lang getrocknet. Das getrocknete Polymerisat wurde grob zerstoßen, gemahlen und auf ein Pulver mit einer Partikelgröße von 150 bis 850 µm gesiebt.

Zur Nachvernetzung wurden 100g des oben erhaltenen Pulvers unter kräftigen Rühren mit einer Lösung aus 1 g 1,3-Dioxalan-2-on, 3g Wasser und 0,5 g Aluminiumsulfat-18-Hydrat vermischt und anschließend für 40 min in einem Ofen, der auf 180°C temperiert war, erhitzt.

### Beispiel 2: Herstellung eines biologisch abbaubaren Polymers

Das im Beispiel 1 erhaltene, nachvernetzte Polymer wurde mit einer wasserlöslichen Weizenstärke (dem Produkt Foralys^{®}380 der Firma Roquette, Lestrem, Frankreich) im Gewichtsverhältnis Polymer: Stärke von 4: 1 unter trockenen Bedingungen gemischt und anschließend für 45 Minuten in einem Rollenmixer Typ BTR 10 der Firma Fröbel GmbH, Deutschland, weiter homogenisiert.

Das Produkt wies eine biologische Abbaubarkeit gemäß dem modifizierten Sturmtest nach 28 Tagen von 40 % und einen CRC-Wert von 30,0 g/g auf Der Nachhaltigkeitsfaktor betrug etwa 99.

### BEZUGSZEICHENLISTE

- 1: Bioreaktor
- 2: Zuführung für zu spaltendes organisches Material
- 3: Erste Aufreinigungsvorrichtung
- 4: Eduktvorlage
- 5: Verdampfer
- 6: Katalysatorbett des Dehydratisierungsreaktor
- 7: Kondensationsvorrichtung
- 8: Zuleitung für ein Lösungsmittel
- 9: Produktauslass zur weiteren Aufarbeitung der Acrylsäure
- 10: Heizelement
- 11: Pumpe

## Patentansprüche

1. Verfahren zur Herstellung von Polymeren durch radikalische Polymerisation von Acrylsäure, wobei das Polymer ein wasserabsorbierendes Polymer ist, welches erhältlich ist durch ein Verfahren umfassend die folgenden Verfahrensschritte:
i) Polymerisation der Acrylsäure in Gegenwart eines Vernetzers unter Ausbildung eines Polymergels;
ii) gegebenenfalls Zerkleinerung des Polymergels;
iii) Trocknung des gegebenenfalls zerkleinerten Polymergels unter Erhalt wasserabsorbierender Polymergebilde, sowie
iv) gegebenenfalls Oberflächennachbehandlung der wasserabsorbierenden Polymergebilde,
wobei die Acrylsäure durch ein Syntheseverfahren erhalten wurde, welches folgenden Verfahrensschritt umfasst:
Spaltung eines organischen Materials mittels eines Enzyms oder mindestens eines Bestandteils von einem Enzym, durch Fermentation oder durch aus Zellen isolierten, gegebenenfalls auf einem Substrat immobilisierten Enzymen oder mindestens eines Bestandteils von Enzymen erfolgt, das organische Material zu mindestens 75 Gew.-% auf einem Kohlenhydrat basiert, wobei als organisches Material Malzextrakt, Hafermehl oder Milchpulver, reine, definierte Kohlenhydrate, insbesondere Polysaccharide, wie Raffinose, Stärke, Zellulose, Glykogen oder Dextrine, Disaccharide, wie Saccharose, Laktose oder Maltose, und Monosaccharide, vorzugsweise Hexosen wie Galaktose, Xylose, Glukose, Galaktose oder Fruktose, oder aber Zuckeralkohole eingesetzt werden, wobei durch die Spaltung des Kohlenhydrates β-Hydroxypropionsäure erhalten wird und
wobei das Syntheseverfahren zur Herstellung der Acrylsäure neben der enzymatischen Spaltung des Kohlenhydrates unter Erhalt von β-Hydroxypropionsäure als Verfahrensschritt a) und einen Verfahrensschritt b) umfasst:
- katalytische Dehydratisierung der β-Hydroxypropionsäure unter Erhalt von Acrylsäure
wobei als Katalysator ein mit einer anorganischen Säure in Kontakt gebrachter, porenförmiger Trägerkörper eingesetzt wird und der porenförmige Trägerkörper zu mindestens 90 Gew.-% auf einem Siliziumoxid basiert und eine Oberfläche in einem Bereich von 0,005 bis 450 m²/g aufweist.

2. Verfahren nach Anspruch 1, wobei es sich bei der anorganischen Säure um Phosphorsäure handelt.

3. Verfahren nach einem der Ansprüche 1 oder 2 wobei die Dehydratisierung der β-Hydroxypropionsäure in flüssiger Phase oder in der Gasphase erfolgt.

4. Verfahren nach einem der Ansprüche 1 oder 2, wobei mindestens 80 % der Acrylsäure durch das Syntheseverfahren umfassend den Verfahrensschritt a) erhalten wurden.

## Claims

1. Process for production of polymers by free-radical polymerization of acrylic acid wherein the polymer is a water-absorbing polymer obtainable by a process comprising the steps of:
i) polymerizing the acrylic acid in the presence of a crosslinker to form a polymer gel;
ii) optionally comminuting the polymer gel;
iii) drying the optionally comminuted polymer gel to obtain water-absorbing polymer structures; and
iv) optionally surface-aftertreating the water-absorbing polymer structures;
wherein the acrylic acid was obtained by a method of synthesis comprising the step of:
splitting an organic material by means of an enzyme or of at least one constituent of an enzyme, through fermentation or through enzymes isolated from cells and optionally immobilized on a substrate, or at least one constituent of enzymes, the organic material is at least 75 wt% based on a carbohydrate, wherein the organic material used comprises malt extract, oatmeal or milk powder, pure defined carbohydrates, in particular polysaccharides, such as raffinose, starch, cellulose, glycogen or dextrins, disaccharides, such as sucrose, lactose or maltose, and monosaccharides, preferably hexoses such as galactose, xylose, glucose, galactose or fructose, or else sugar alcohols, wherein the step of splitting the carbohydrate gives β-hydroxypropionic acid and wherein the method of synthesis for the acrylic acid, in addition to a step a) of enzymatically splitting the carbohydrate to obtain β-hydroxypropionic acid, comprises a step b) of:
- catalytically dehydrating the β-hydroxy-propionic acid to obtain acrylic acid,
wherein the catalyst used is a porous supporting body contacted with an inorganic acid and the porous supporting body is at least 90 wt% based on a silicon oxide and has a surface area in a range extending from 0.005 to 450 m²/g.

2. Process according to Claim 1, wherein the inorganic acid comprises phosphoric acid.

3. Process according to either of Claims 1 and 2, wherein the step of dehydrating the β-hydroxypropionic acid is effected in the liquid phase or in the gas phase.

4. Process according to either of Claims 1 and 2, wherein at least 80% of the acrylic acid was obtained by the method of synthesis comprising step a).

## Revendications

1. Procédé pour la préparation de polymères par polymérisation radicalaire d'acide acrylique, le polymère étant un polymère absorbant l'eau, qui peut être obtenu par un procédé comprenant les étapes de processus suivantes :
i) polymérisation de l'acide acrylique en présence d'un agent de réticulation, avec formation d'un gel polymère ;
ii) éventuellement fragmentation du gel polymère ;
iii) séchage du gel polymère éventuellement fragmenté, avec obtention de produits polymères absorbant l'eau, ainsi que
iv) le cas échéant traitement de surface des produits polymères absorbant l'eau,
l'acide acrylique ayant été obtenu par un procédé de synthèse qui comprend l'étape de processus suivante :
coupure d'une matière organique au moyen d'une enzyme ou d'au moins un composant d'une enzyme, effectuée par fermentation ou par des enzymes ou au moins un composant d'enzymes, isolées à partir de cellules, éventuellement immobilisées sur un support, la matière organique étant à raison d'au moins 75 % en poids à base d'un glucide, en utilisant comme matière organique de l'extrait de malt, de la farine d'avoine ou du lait en poudre, des glucides définis, purs, en particulier des polysaccharides, tels que le raffinose, l'amidon, la cellulose, le glycogène ou des dextrines, des disaccharides, tels que le saccharose, le lactose ou le maltose, et des monosaccharides, de préférence des hexoses tels que le galactose, le xylose, le glucose, le galactose ou le fructose, ou bien des alcools glucidiques, de l'acide β-hydroxypropionique étant obtenu par la coupure du glucide et le procédé de synthèse pour la préparation de l'acide acrylique comprenant, outre la coupure enzymatique du glucide avec obtention d'acide β-hydroxypropionique en tant qu'étape a) de processus, une étape b) de processus :
- déshydratation catalytique de l'acide β-hydroxypropionique avec obtention d'acide acrylique,
en utilisant comme catalyseur un corps de support poreux mis en contact avec un acide inorganique et le corps de support poreux étant à raison d'au moins 90 % en poids à base d'un oxyde de silicium et présentant une surface dans la plage de 0,005 à 450 m²/g.

2. Procédé selon la revendication 1, dans lequel pour ce qui est de l'acide inorganique il s'agit d'acide phosphorique.

3. Procédé selon l'une quelconque des revendications 1 et 2, dans lequel la déshydratation de l'acide β-hydroxypropionique s'effectue en phase liquide ou en phase gazeuse.

4. Procédé selon l'une quelconque des revendications 1 et 2, dans lequel au moins 80 % de l'acide acrylique ont été obtenus par le procédé de synthèse comprenant l'étape a) de processus.
